# EUROPEAN PATENT APPLICATION

(11) **EP 1 444 986 A1**
(43) Date of publication of application: **11.08.2004**
(21) Application number: 03004900.1
(22) Date of filing: 06.03.2003
(51) Int. Cl.: A61K 38/37, A61K 38/38, A61P 7/04

(54) **Pharmaceutical preparation for the improved treatment of blood-clotting disorders**

(30) Priority: 07.02.2003 EP 03002598
(71) Applicant: Aventis Behring GmbH, 35002 Marburg (DE)
(72) Inventor: Hauser, Hans-Peter, Dr., 35041 Marburg (DE); Weimer, Thomas, Dr., 35075 Gladenbach (DE); Kronthaler, Ulrich, Dr., 35051 Marburg (DE)

(57) **Abstract**

The present invention relates to a pharmaceutical preparation for the improved treatment of blood-clotting disorders containing one or several factor VIII derived peptides which increases the plasma half life of factor VIII. Further, such peptides, their DNA-sequences, their recombinant expression as albumin fusion proteins and vectors containing such DNA-sequences as well as host cells for such recombinant expression vectors and the use of such DNA-sequences in a transfer vector for gene therapy are disclosed.

## Description

The present invention relates to a pharmaceutical preparation for the improved treatment of blood-clotting disorders containing one or several factor VIII derived peptides which increases the plasma half life of factor VIII. Further, such peptides, their DNA-sequences, their recombinant expression as albumin fusion proteins and vectors containing such DNA-sequences as well as host cells for such recombinant expression vectors and the use of such DNA-sequences in a transfer vector for gene therapy are disclosed.

Classic haemophilia or haemophilia A is the most common of the inherited bleeding disorders. The clinical manifestation of haemophilia A is an abnormal bleeding tendency and before treatment with factor VIII concentrates was introduced the mean life span for a person with severe haemophilia was less than 20 years. The use of concentrates of factor VIII from plasma has considerably improved the situation for the haemophilia patients. The mean life span has increased extensively, giving most of them the possibility to live a more or less normal life. However, there have been certain problems with the plasma derived concentrates and their use, the most serious of which have been the transmission of viruses. So far, viruses causing AIDS, hepatitis B, and non A non B hepatitis have hit the population seriously. Although different virus inactivation methods and new highly purified factor VIII concentrates have recently been developed an inadvertent contamination can not be excluded. Also, the factor VIII concentrates are fairly expensive because the limited supply of human plasma raw material.

A factor VIII product derived from recombinant material is likely to solve a large extent of the problems associated with the use of plasma derived factor VIII concentrates for treatment for haemophilia A. However, the development of a recombinant factor VIII has met with some difficulties, for instance the problem of achieving production levels in sufficiently high yields, in particular regarding the full-length molecule.

In fresh plasma prepared in the presence of protease inhibitors, factor VIII has been shown to have a molecular weight of 280 kDa and to be composed of two polypeptide chains of 200 kDa and 80 kDa, respectively. These chains are held together by metal ion bridges. More or less proteolytically degraded forms of the factor VIII molecule can be found as active fragments in factor VIII material purified from commercial concentrates. The fragmented form of factor VIII having molecular weights from 260 kDa down to 170 kDa, consists of one heavy chain with a molecular weight ranging from 180 kDa down to 90 kDa, where all variants have identical amino termini, in combination with one 80 kDa light chain. The amino-terminal region of the heavy chain is identical to that of the single chain factor VIII polypeptide that can be deduced from the nucleotide sequence data of the factor VIII cDNA.

The smallest active form of factor VIII with a molecular weight of 170 kDa, consisting of one 90 kDa and one 80 kDa chain, can be activated with thrombin to the same extent as the higher molecular weight forms, and thus represents an inactivated form. It has also been shown to have full biological activity in vivo as tested in haemophilia dogs. Thus, the haemostatic effectiveness of the 170 kDa form is the same as for the high molecular weight forms of factor VIII.

FVIII is secreted into plasma as a heterodimer of a heavy chain (domains A1-A2-B) and a light chain (A3-C1-C2) associated through a noncovalent divalent metal ion linkage between the A1- and A3 domains. In plasma, FVIII is stabilized by binding to von Willebrand factor.

Upon proteolytic activation by thrombin, FVIII is activated to a heterotrimer of two heavy chain fragments (A1, a 50 kDa fragment, and A2 a 43 kDa fragment) and the light chain (A3-C1-C2, a 73 kDa fragment). The active form of FVIII (FVIIIa) thus consists of an A1-subunit associated through the divalent metal ion linkage to a thrombin-cleaved A3-C1-C2 light chain and a free A2 subunit associated with the A1 domain. The dissociation of that free A2 subunit from the heterotrimer is thought to be the rate limiting step in FVIIIa inactivation after thrombin activation.
Non-activated FVIII has in plasma a half-life of about 14 hours. It is thought that FVIII first binds to heparan sulphate proteoglycans on cell surfaces and then after binding to LRP (low density lipoprotein receptor related protein) is taken up by mainly hepatic cells and catabolised (Ananyeva et al., TCM, Vol. 11, No. 6 (2001) 251 - 257). The contact sites to LRP within FVIII were localized to the A2_site (residues 484 - 509; SEQ ID No. 5) (Saenko et al., J. Biol. Chem. 274 (1999), 37, 685 - 692) and the C2 domain (Lenting et al (1999) J. Biol. Chem., 274; 23, 734-739). Blocking LRP by RAP (receptor associated protein) was shown to increase FVIII levels (Schwarz et al (2000) Blood 95: 1.703 - 1.708). It has been suggested to use RAP to block LRP in order to increase FVIII plasma half life (WO 00/27425) or to vary the FVIII sequence in order to prevent binding to LRP also to increase FVIII half-life (WO 00/28021 and WO 00/71714).

Due to the plasma half life of 14 hours FVIII has to be administered i.v. to haemophilia patients who are on prophylactic treatment about 3 times per week. It would thus be highly desirable to create a FVIII with enhanced plasma half life which could lead to a FVIII preparation which has to be administered less frequently. The present invention offers a solution to this problem by blocking the receptors responsible for FVIII catabolism by fragments of FVIII attaching to these receptors or by these fragments fused to human serum albumin.

Subject of this invention is therefore a pharmaceutical preparation to improve the treatment of blood clotting disorders, which contains a peptide which comprises one or several binding sites for the heparan sulphate proteoglycan (HSPG) and/or the low density lipoprotein receptor related protein (LRP) of the human factor VIII sequence. Such peptides can be stabilized by fusing or by chemically attaching them to larger plasma proteins with enhanced plasma half-life, preferably to albumin.

In addition to such a peptide or an albumin fusion protein which comprises one or several binding sites for heparan sulphate proteoglycan (HSPG) and/or low density lipoprotein receptor related protein (LRP) of the human factor VIII sequence said pharmaceutical preparation may contain a wild-type factor VIII or a coagulation active mutant of factor VIII. Alternatively FVIII is administered separately.

Such fusion proteins or peptides impair the degradation of factor VIII in plasma and, consequently, increase the half life of factor VIII in plasma because of their capacity to competitively bind to HSPG and/or LRG. The reduction of the plasma concentration of said factor VIII-binding compounds reduces the degradation of factor VIII.

The albumin fusion proteins or peptides which are used according to this invention as an ingredient of a pharmaceutical preparation comprise the amino acids 558-565 and/or 484 - 509 of the A2-subunit of the human factor VIII sequence and correspond to the amino acid sequence of SEQ ID No. 1 - No. 6. of the attached sequence listing.

Fusing therapeutic proteins to human serum albumin has been shown a means to increase plasma half life (WO 01/77137 . However, it was unclear whether the LRP binding fragment (residue 484 - 509) from FVIII would in an albumin fusion be present in a conformation still allowing the binding to LRP.

As FVIII binding to LRP is most probably a two step process of first binding to heparan sulphate proteoglycans (HSPG) via residues 558 - 565 (SEQ ID No. 6) and only then to LRP (Ananyeva et al., TCM, Vol. 11, No. 6 (2001) 251 - 257) a fusion protein displaying only the LRP binding site (SEQ ID No. 1) was compared to a fusion protein containing both the LRP and the HSPG binding site (SEQ ID No. 2).

In order to enable cleavage of the fusion proteins upon FVIII activation by thrombin, a thrombin cleavage site was inserted between the albumin and the FVIII fragment portion in a second set of fusion proteins (SEQ ID No. 3 and 4).

The present invention further comprises a recombinant expression vector containing any of the DNA-sequences disclosed herein as well as host cells being transformed with any of said recombinant expression vectors and the use of said DNA-sequences in a transfer vector for the human gene therapy.

Further details of the invention are shown in the following examples:

### Examples

### Cloning of LRP binding site

LRP binding site and flanking amino acid (amino acids 479 - 517, nomenclature as in Ananyeva et al., TCM, Vol. 11, No. 6 (2001) 251 - 257) was cloned into pLITMUS29 (New England Biolabs) after amplification on a FVIII cDNA containning plasmid using primers We1003 ^{5'}GTGGGATCCGGTGGTGGA ATCACTGATGTCCGTCC^{3'} (=SEQ. ID No. 7) and We1059 ^{5'}CACAAGCTT ATTATACAGTCACTGTCCATTTATATTTG^{3'} (=SEQ. ID No. 8) (restriction sites used for coning are underlined), subsequent restriction digest with BamH1 and HindIII, purification and ligation into BamH1/HindIII cut pLITMUS29. The resulting plasmid was designated pCN175.

### Cloning of LRP/HSPG binding site

LRP and HSPG binding site and flanking amino acids (amino acids 479 - 575, nomenclature as in Ananyeva et al., TCM, Vol. 11, No. 6 (2001) 251 - 257) was cloned into pLITMUS29 after amplification on a FVIII cDNA containing plasmid using primers We1003 ⁵'GTGGGATCCGGTGGTGGAATCACTCATGTCCG TCC^{3'} (=SEQ. ID No. 7) and We1004 ^{5'}CACAAGCTTATTACAGGATGA CA TTCCTCTTGTC^{3'} (=SEQ. ID No. 9) (restriction sites used for cloning are underlined), subsequent restriction digest with BamH1 and HindIII, purification and ligation into BamH1/HindIII cut pLITMUS29. The resulting plasmid was designated pGH157.

After clone isolation and sequence verification LRP and LRP/HSPG fragments, isolated after BamH1/HindIII digestion and purification from preparative gels, were inserted into an expression vector for C-terminal albumin fusion.

### Cloning into yeast expression vectors

Plasmid pDB2243, described by Sleep, D., et al. (1991) Bio/Technology 9, 183-187 which contained the yeast PRB1 promoter and the yeast ADH1 terminator providing appropriate transcription terminator sequences as well as the human albumin coding sequence, was used. First, plasmid pDB2243 was digested to completion with BamHI, the recessed ends were blunt ended with T4 DNA polymerase and dNTPs, and finally religated to generate plasmid pDB2566. Second, a Glycin-Serin-linker [(GGs)₄GG] was inserted at the C-terminus of the albumin sequence. For that pDB2566 was digested with Bsu36I and HindIII, and two linker oligonucelotides (GSL+, TTAGGCTTAGGTGGTTCTGGTGGTTCCGGTGGTTCTGGTGGATCC GGTGGA^{3'} (=SEQ. ID No. 10). GSL-, ^{5'}AGCTTCCACCGGATCCACCAGAAC CACCGGAACCACCAGAACCACCTAAGCC^{3'} (=SEQ. ID No. 11) encompassing the (GGS)₄GG linker and a BamH1 cloning site were inserted. The resulting plasmid was designated pDB2566GS.

The LRP and LRP/HSPG BamH1/HindIII DNA fragments were isolated from the plasmids described above and ligated with plasmid pDB2566GS, which had partially been digested with Hindlll and then digested to completion with BamHI. Resulting plasmid pCN176 contained the LRP, plasmid pGH159 contained the LRP/HSPG fragment.

Appropriate yeast vector sequences were provided by a "disintegration" plasmid pSAC35 generally disclosed in EP-A-286 424 and described by Sleep, D., et al. (1991) Bio/Technology 9, 183 - 187. The Notl albumin-(GGS)₄GG-LRP expression cassette was isolated from plasmid pCN176, purified and ligated into Notl digested pSAC35 which had been treated with calf intestinal phosphatase, creating two plasmids; the first pCN177 contained the Notl expression cassette in the same expression orientation as LEU2 and was used for expression of the albumin fusion protein, while the second contained the Notl expression cassette in the opposite orientation to LEU2.

The Notl albumin-(GGS)₄GG.LRP/HSPG expression cassette was isolated from plasmid pGH159, purified and ligated into Notl digested pSAC35 which had been treated with calf intestinal phosphatase, creating two plasmids; the first pGH165 contained the Notl expression cassette in the same expression orientation as LEU2 and was used for expression of the albumin fusion protein, while the second contained the Notl expression cassette in the opposite orientation to LEU2.

### Introduction of thrombin cleavage sites

A thrombin cleavage site (TCS) was introduced between albumin and the LRP/HSPG and LRP sites, respectively, by inserting a linker into the respective BamH1 sites of plasmids pCN176 and pGH159. For that the plasmids pCN176 and pGH159 were linearized with BamH1 and, after dephosphorylation, ligated together with a hybridized linker consisting of 5'-phosphorylated oligonucleotides ^{5'}GATCTCTAGTTCCACGTG ^{3'} (=SEQ. ID No. 12) and ^{5'}GATCCACGTGGAACTAGA^{3'} (=SEQ. ID No. 13). Resulting plasmids with the linker in correct orientation (see figure below =SEQ. ID No. 14 and 15) were designated pCN167 containing the LRP, and pGH168, containing the LRP/HSPG fragments.

SEQ. ID No. 14 and 15
...GCC TTA GGC TTA GGT GGT TCT GGT GGT TCC GGT GGT TCT GGT GGA TCT CTA GTT CCA CGT
...Ala Leu Gly Leu Gly Gly Ser Gly Gly Ser Gly Gly Ser Gly Gly Ser Leu Val Pro Arg
...      ............rHA.... <<                                                    >>............TCS...
                    >>                                    GGS Linker .........<<

thrombin cleavage site
↓
GGA TCC GGT GGT GGA ATC ACT GAT GTC CGT CCT TTG TAT TCA AGG AGA TTA CCA AAA..
Gly Ser Gly Gly Gly Ile Thr Asp Val Arg Pro Leu Tyr Ser Arg Arg Leu Pro Lys...
............TCS.........<<
>>.........................................................FVIII LRP binding site...............................................

Appropriate yeast vector sequences were provided by plasmid pSAC35 as described above. The Notl albumin(GGS)₄-TCS-LRP expression cassette was isolated from plasmid pCN167, purified and ligated into Notl digested pSAC35 which had been treated with calf intestinal phosphatase, creating two plasmids; the first pCN169 contained the Notl expression cassette in the same expression orientation as LEU2 and was used for expression of the albumin fusion protein, while the second contained the Notl expression cassette in the opposite orientation to LEU2.

The Notl albumin-(GGS)₄-TCS-LRP/HSPG expression cassette was isolated from plasmid pGH168, purified and ligated into Notl digested pSAC35 which had been treated with calf intestinal phosphatase, creating two plasmids; the first pGH171 contained the Notl expression cassette in the same expression orientation as LEU2 and was used for expression, while the second contained the Notl expression cassette in the opposite orientation to LEU2.

### Transformation of yeast

Yeast strains disclosed in WO 95/23857, WO 95/33833 and WO 94/04687 were transformed to leucine prototrophy. The transformants were patched out onto Buffered Minimal Medium and incubated at 30°C until grown sufficiently for further analysis.

### Expression and purification of albumin fusion proteins

rHA fusions were expressed in shake flask culture and the expression levels were monitored. The albumin fusion proteins were purified by cation exchange and gel permeation chromatography. The purified material was then concentrated and diafiltered against PBS.

### Pharmacodynamic/pharmacokinetic studies

The primary objective of the combined pharmacodynamic/pharmacokinetic study is to generate proof for the increase half-life of FVIII, mediated by blocking its uptake and degradation into compartments different from plasma.

This objective will be achieved by assessing the modulation of pharmacodynamic/pharmacokinetic parameters of FVIII by AFP X (whereas X represents any of the LRP/HSPG albumin fusion proteins described above). As endogenous levels of FVIII might flaw the selected endpoint, the study will be performed in FVIII ko mice. As presumable only one sample per mouse can be collected the study is split into two parts. The first will lead to a suitable dose level of AFP X, while the second will scrutinize on the modulation of the excretion profile by the selected dose level.

### Primary objective:

To assess the half-life and bioavailability of FVIII versus FVIII co-infused with LRP albumin-fusion-protein X, by means of determining plasma levels and by determining haemostasis in the tail cut bleeding model. Bleeding time assessment might deliver a more accurate reflection of FVIII levels as compared to antigen levels.

### Secondary objective:

To potentially assess additional pharmacokinetic parameters of FVIII versus FVIII coinfused with LRP fusion-protein X.

### Study design

**Table 1:**

| **Treatment groups - fixed timepoint** | | | |
|---|---|---|---|
| **No.** | **Treatment** | **Dose / schedule / route** | **n (m/f)** |
| 1 | FVIII | 50 U/kg / single injection / i.v. | 5 m + 5 f |
| 2 | AFP X | 10 mg/kg / single injection / i.v. | 5 m + 5 f |
| 3 | FVIII + | 50 U/kg / single injection / i.v. + | 5 m + 5 f |
| | AFP X | 10 mg/kg / single injection / i.v. | |
| 4 | FVIII + | 50 U/kg / single injection / i.v. + | 5 m + 5 f |
| | AFP X | 100 mg/kg / single injection / i.v. | |
| 5 | FVIII + | 50 U/kg / single injection / i.v. + | 5 m + 5 f |
| | AFP X | 500 mg/kg /single injection / i.v. | |

**Table 2:**

| **Treatment groups - fixed dose** | | | | |
|---|---|---|---|---|
| **No.** | **Treatment** | **Dose / schedule / route** | **Sampling timepoint** | **n (m/f)** |
| 1 | FVIII | 50 U/kg / single injection / i.v. | 5 hours | 5 m + 5 f |
| 2 | AFP X | x mg/KG / single injection / i.v. | 5 hours | 5 m + 5 f |
| 3 | FVIII + | 50 U/kg / single injection / i.v. + | 5 hours | 5 m + 5 f |
| | AFP X | x mg/kg / single injection / i.v. | | |
| 4 | FVIII | 50 U/kg / single injection / i.v. | 1 day | 5 m + 5 f |
| 5 | AFP X | x mg/kg / single injection / i.v. | 1 day | 5 m + 5 f |
| 6 | FVIII + | 50 U/kg / single injection / i.v. + | 1 day | 5 m + 5 f |
| | AFP X | x mg/kg / single injection / i.v. | | |

### Materials and Methods

### Administration of test article (1.-2.):

| | |
|---|---|
| Test article 1 | AFP X |
| Manufacturer | Aventis Behring GmbH, Lab Thomas Weimer |
| Batch No. | tbd |
| Application volume | tbd |
| Single dose/route | 10, 100 or 500 mg/kg, i.v. |
| Frequency | 1 × (t = 0) |

| | |
|---|---|
| Test article 2 | Helixate or a suitable other FVIII concentrate |
| Manufacturer | Aventis Behring GmbH |
| Batch No. | tbd |
| Application volume | tbd |
| Single dose/route | 50 U/kg, i.v. |
| Frequency | 1 × (t = 0) |

### Experimental Animals

| | |
|---|---|
| Species /Strain | C57BI6 Mouse, FVIII-ko |
| Sex/Age | 55 male / 55 female |
| No. total | 110 |
| Supplier | Charles River, Kisslegg |

### Animal model:

Five male and five female FVIII ko mice per group will receive FVIII alone or in combination with AFP X at three dose levels (10, 100 or 500 mg/kg) by a single i.v. injection on day 0.

Blood samples are to be drawn for the determination of the respective antigen or activity levels at the appropriate timepoints.

### Study Variables:

Pharmacokinetic (PK) variables, depending on the frequency of samples:
Half-lives, area under the plasma concentration time curve up to the last plasma sample (AUC_{0-and}), maximum concentration (Cₘₐₓ), time to maximum concentration (tₘₐₓ).

Pharmacodynamic endpoints:
Tail cut bleeding time, blood loss with cut off value of 10 min. The bleeding time will be determined in anaesthesia.

### Statistical methods:

### Analysis of individual plasma levels

The plasma concentration-time profiles of FVIII and potentially AFP X following i.v. injection will analyzed per animal by means of non-linear regression, if applicable. An exponential model may be fitted to the baseline-adjusted data by the method of least squares.

The AUC may be calculated using the linear trapezoidal rule up to the last measured value (AUC _{0-end}).

### Summary and comparative analyses

Individual PK results will summarized descriptively per treatment (minimum, median, maximum, mean, standard deviation), completed by a two-way analysis of variance was carried out for the following variables: elimination half-life, AUC and Cₘₐₓ (all log-transformed). Treatment group may be a fixed factor. Appropriate contrasts between treatment groups should be evaluated.

### Description of SEQ-IDs

**SEQ ID No.1** represents the LRP binding site fused to human albumin. 1-24, leader sequence, which is removed during secretion in yeast; 25-609, human albumin; 610-623, GGS linker; 624-662, LRP binding site (629-654) and additional FVIII derived flanking sequences (624-628; 655-662).
**SEQ ID No.2** represents the LRP/HSPG binding sites fused to human albumin. 1-24, leader sequence, which is removed during secretion in yeast; 25-609, human albumin; 610-623, GGS linker; 624-720, LRP and HSPG binding sites within the FVIII context; LRP binding site (629-654), HSPG binding site (703-710).
**SEQ ID No.3** represents the LRP binding site fused to human albumin, including an additional thrombin cleavage site. 1-24, leader sequence, which is removed during secretion in yeast; 25-609, human albumin; 610-621, GGS linker; 622-668, LRP binding site (635-660) and additional FVIII derived flanking sequences (630-634; 661-668); 625/626, thrombin cleavage site.
**SEQ ID No.4** represents the LRP/HSPG binding sites fused to human albumin, including an additional thrombin cleavage site. 1-24, leader sequence, which is removed during secretion in yeast; 25-609, human albumin; 610-621, GGS linker; 630-726, LRP and HSPG binding sites within the FVIII context; LRP binding site (635-660), HSPG binding site (709-716); 625/626, thrombin cleavage site.
**SEQ ID No.5** shows the contact sites to LRP within the Factor VIII A2 domain, amino acids residues 484 - 509.
**SEQ ID No.6** shows the HSPG binding sites within the Factor VIII A2 domain, amino acids residues 558 - 565.
**SEQ ID No.7 to 9** represent synthetic oligonucleotides used for cloning the LRP and LRP/HSPG binding sites.
**SEQ ID No.10 and 11** represent synthetic oligonucleotides used for modification of the yeast vector pDB2243.
**SEQ ID No.12 and 13** represent synthetic oligonucleotides used for introduction of a thrombin cleavage site.
**SEQ ID No. 14 and 15** display the junction of albumin and LRP binding site with inserted GGS linker and thrombin cleavage site within plasmids pCN167 and pGH168.

## Claims

1. Pharmaceutical preparation to improve the treatment of blood-clotting disorders, **characterised in that** it contains a peptide which comprises one or several binding sites for the heparan sulphate proteoglycan (HSPG) and/or the low density lipoprotein receptor related protein (LRP) of the human factor VIII sequence.

2. Pharmaceutical preparation as claimed in claim 1, **characterised in that** it contains in addition to the peptide comprising one or several binding sites for heparan sulphate proteoglycan (HSPG) and/or low density lipoprotein receptor related protein (LRP) of the human factor VIII sequence a wild-type factor VIII or a coagulation active mutant of factor VIII.

3. Peptide **characterised in that** it contains one or several binding sites for heparan sulphate proteoglycan (HSPG) and/or low density lipoprotein receptor related protein (LRP) of the human factor VIII sequence.

4. Peptide as claimed in claim 3, **characterised in that** it contains the amino acids 558 - 565 and/or 484 - 509 of the A2-subunit of the human factor VIII sequence.

5. Albumin fusion protein, **characterised in that** it contains one ore several binding sites for heparan sulphate proteoglycan (HSPG) and/or low density lipoprotein receptor related protein (LRP) of the human factor VIII sequence.

6. Albumin fusion protein as claimed in claim 5, **characterised in that** it contains the amino acids 558 - 565 and/or 484 - 509 of the A2-subunit of the human factor VIII sequence.

7. Albumin fusion protein as claimed in claims 5 and 6, **characterised in that** it contains a thrombin cleavage site between the albumin sequence and the human factor VIII sequence.

8. Albumin fusion protein as claimed in claims 5 to 7, **characterised in that** it contains the amino acid sequence of SEQ ID No.1, SEQ ID No.2, SEQ ID No.3 or SEQ ID No.4 of the attached sequence listing.

9. DNA sequence coding for any of the amino acid sequences of claims 3 to 8.

10. Recombinant expression vector containing any of the DNA sequences of claim 9.

11. Host cells being transformed with a recombinant expression vectors of claim 10.

12. Use of any of the DNA sequences of claim 9 in a transfer vector for the human gene therapy.


SEQUENCE LISTING

<110>  Aventis Behring GmbH

<120>  Pharmaceutical Preparation for the Improved Treatment of Blood-
Clotting Disorders

<130>  C9 P 84 /A56

<160>  13

<170>  PatentIn version 3.1

<210>  1

<211>  662

<212>  PRT

<213>  Homo sapiens

<400>  1

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala

515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Gly Gly Gly
    610                 615                 620

Ile Thr Asp Val Arg Pro Leu Tyr Ser Arg Arg Leu Pro Lys Gly Val
625                 630                 635                 640

Lys His Leu Lys Asp Phe Pro Ile Leu Pro Gly Glu Ile Phe Lys Tyr
                645                 650                 655

Lys Trp Thr Val Thr Val
            660

<210>  2

<211>  720

<212>  PRT

<213>  Homo sapiens

<400>  2

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala

20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala

            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Gly Gly Gly

    610                 615                 620

Ile Thr Asp Val Arg Pro Leu Tyr Ser Arg Arg Leu Pro Lys Gly Val
625                 630                 635                 640

Lys His Leu Lys Asp Phe Pro Ile Leu Pro Gly Glu Ile Phe Lys Tyr
                645                 650                 655

Lys Trp Thr Val Thr Val Glu Asp Gly Pro Thr Lys Ser Asp Pro Arg
            660                 665                 670

Cys Leu Thr Arg Tyr Tyr Ser Ser Phe Val Asn Met Glu Arg Asp Leu
        675                 680                 685

Ala Ser Gly Leu Ile Gly Pro Leu Leu Ile Cys Tyr Lys Glu Ser Val
    690                 695                 700

Asp Gln Arg Gly Asn Gln Ile Met Ser Asp Lys Arg Asn Val Ile Leu
705                 710                 715                 720

<210>  3

<211>  668

<212>  PRT

<213>  Homo sapiens

<400>  3

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Leu Val Pro
    610                 615                 620

Arg Gly Ser Gly Gly Gly Ile Thr Asp Val Arg Pro Leu Tyr Ser Arg
625                 630                 635                 640

Arg Leu Pro Lys Gly Val Lys His Leu Lys Asp Phe Pro Ile Leu Pro
                645                 650                 655

Gly Glu Ile Phe Lys Tyr Lys Trp Thr Val Thr Val
            660                 665

<210>  4

<211>  726

<212>  PRT

<213>  Homo sapiens

<400>  4

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp

                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Leu Val Pro
    610                 615                 620

Arg Gly Ser Gly Gly Gly Ile Thr Asp Val Arg Pro Leu Tyr Ser Arg
625                 630                 635                 640

Arg Leu Pro Lys Gly Val Lys His Leu Lys Asp Phe Pro Ile Leu Pro
                645                 650                 655

Gly Glu Ile Phe Lys Tyr Lys Trp Thr Val Thr Val Glu Asp Gly Pro
            660                 665                 670

Thr Lys Ser Asp Pro Arg Cys Leu Thr Arg Tyr Tyr Ser Ser Phe Val
        675                 680                 685

Asn Met Glu Arg Asp Leu Ala Ser Gly Leu Ile Gly Pro Leu Leu Ile
    690                 695                 700

Cys Tyr Lys Glu Ser Val Asp Gln Arg Gly Asn Gln Ile Met Ser Asp
705                 710                 715                 720

Lys Arg Asn Val Ile Leu
                725

<210>  5

<211>  26

<212>  PRT

<213>  Homo sapiens

<400>  5
Arg Pro Leu Tyr Ser Arg Arg Leu Pro Lys Gly Val Lys His Leu Lys
1               5                   10                  15

Asp Phe Pro Ile Leu Pro Gly Glu Ile Phe
            20                  25

<210>  6

<211>  8

<212>  PRT

<213>  Homo sapiens

<400>  6
Ser Val Asp Gln Arg Gly Asn Gln
1               5

<210>  7

<211>  35

<212>  DNA

<213>  Homo sapiens

<400>  7
GTGGGATCCG GTGGTGGAAT CACTGATGTC CGTCC                                35

<210>  8

<211>  38

<212>  DNA

<213>  Homo sapiens

<400> 8
CACAAGCTTA TTATACAGTC ACTGTCCATT TATATTTG 38

<210> 9

<211> 34

<212> DNA

<213> Homo sapiens

<400> 9
CACAAGCTTA TTACAGGATG ACATTCCTCT TGTC 34

<210> 10

<211> 51

<212> DNA

<213> Homo sapiens

<400> 10
TTAGGCTTAG GTGGTTCTGG TGGTTCCGGT GGTTCTGGTG GATCCGGTGG A 51

<210> 11

<211> 52

<212> DNA

<213> Homo sapiens

<400> 11
AGCTTCCACC GGATCCACCA GAACCACCGG AACCACCAGA ACCACCTAAG CC 52

<210> 12

<211> 18

<212> DNA

<213> Homo sapiens

<400> 12
GATCTCTAGT TCCACGTG 18

<210> 13

<211> 18

<212> DNA

<213> Homo sapiens

<400> 13
GATCCACGTG GAACTAGA 18

<210> 14

<211> 117

<212> DNA

<213> Homo sapiens

<400> 14
GCCTTAGGCT TAGGTGGTTC TGGTGGTTCC GGTGGTTCTG GTGGATCTCT AGTTCCACGT 60

GGATCCGGTG GTGGAATCAC TGATGTCCGT CCTTTGTATT CAAGGAGATT ACCAAAA 117

<210> 15

<211> 39

<212> PRT

<213> Homo sapiens

<400> 15

Ala Leu Gly Leu Gly Gly Ser Gly Gly Ser Gly Gly Ser Gly Gly Ser
1               5                   10                  15

Leu Val Pro Arg Gly Ser Gly Gly Gly Ile Thr Asp Val Arg Pro Leu
            20                  25                  30

Tyr Ser Arg Arg Leu Pro Lys
        35

SEQUENCE LISTING

<110> Aventis Behring GmbH

Aventis Behring GmbH

<120> Pharmaceutical Preparation for the Improved Treatment of Blood-Clotting Disorders

<130> 2003/M002J

<160> 15

<170> PatentIn version 3.1

<210> 1

<211> 662

<212> PRT

<213> Homo sapiens

<400> 1

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
Seite 3

580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Gly Gly Gly
    610                 615                 620

Ile Thr Asp Val Arg Pro Leu Tyr Ser Arg Arg Leu Pro Lys Gly Val
625                 630                 635                 640

Lys His Leu Lys Asp Phe Pro Ile Leu Pro Gly Glu Ile Phe Lys Tyr
                645                 650                 655

Lys Trp Thr Val Thr Val
            660

<210>  2

<211>  720

<212>  PRT

<213>  Homo sapiens

<400>  2

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Gly Gly Gly
    610                 615                 620

Ile Thr Asp Val Arg Pro Leu Tyr Ser Arg Arg Leu Pro Lys Gly Val
625                 630                 635                 640

Lys His Leu Lys Asp Phe Pro Ile Leu Pro Gly Glu Ile Phe Lys Tyr
                645                 650                 655

Lys Trp Thr Val Thr Val Glu Asp Gly Pro Thr Lys Ser Asp Pro Arg
            660                 665                 670

Cys Leu Thr Arg Tyr Tyr Ser Ser Phe Val Asn Met Glu Arg Asp Leu
        675                 680                 685

Ala Ser Gly Leu Ile Gly Pro Leu Leu Ile Cys Tyr Lys Glu Ser Val
    690                 695                 700

Asp Gln Arg Gly Asn Gln Ile Met Ser Asp Lys Arg Asn Val Ile Leu
705                 710                 715                 720

<210>  3

<211>  668

<212>  PRT

<213>  Homo sapiens

<400>  3

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp

65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Leu Val Pro
    610                 615                 620

Arg Gly Ser Gly Gly Gly Ile Thr Asp Val Arg Pro Leu Tyr Ser Arg
625                 630                 635                 640

Arg Leu Pro Lys Gly Val Lys His Leu Lys Asp Phe Pro Ile Leu Pro
                645                 650                 655

Gly Glu Ile Phe Lys Tyr Lys Trp Thr Val Thr Val
            660                 665

<210> 4

<211> 726

<212> PRT

<213> Homo sapiens

<400> 4

Met Lys Trp Val Ser Phe Ile Ser Leu Leu Phe Leu Phe Ser Ser Ala
1               5                   10                  15

Tyr Ser Arg Ser Leu Asp Lys Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser

325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu Gly Gly Ser Gly Gly Ser Gly Gly Ser Gly Gly Ser Leu Val Pro
    610                 615                 620

Arg Gly Ser Gly Gly Gly Ile Thr Asp Val Arg Pro Leu Tyr Ser Arg
625                 630                 635                 640

Arg Leu Pro Lys Gly Val Lys His Leu Lys Asp Phe Pro Ile Leu Pro
                645                 650                 655

Gly Glu Ile Phe Lys Tyr Lys Trp Thr Val Thr Val Glu Asp Gly Pro
            660                 665                 670

Thr Lys Ser Asp Pro Arg Cys Leu Thr Arg Tyr Tyr Ser Ser Phe Val
        675                 680                 685

Asn Met Glu Arg Asp Leu Ala Ser Gly Leu Ile Gly Pro Leu Leu Ile
    690                 695                 700

Cys Tyr Lys Glu Ser Val Asp Gln Arg Gly Asn Gln Ile Met Ser Asp
705                 710                 715                 720

Lys Arg Asn Val Ile Leu
                725

<210>  5

<211>  26

<212>  PRT

<213>  Homo sapiens

<400>  5

Arg Pro Leu Tyr Ser Arg Arg Leu Pro Lys Gly Val Lys His Leu Lys
1               5                   10                  15

Asp Phe Pro Ile Leu Pro Gly Glu Ile Phe
            20                  25

<210> 6

<211> 8

<212> PRT

<213> Homo sapiens

<400> 6

Ser Val Asp Gln Arg Gly Asn Gln
1               5

<210> 7

<211> 35

<212> DNA

<213> Homo sapiens

<400> 7
gtgggatccg gtggtggaat cactgatgtc cgtcc 35

<210> 8

<211> 38

<212> DNA

<213> Homo sapiens

<400> 8
cacaagctta ttatacagtc actgtccatt tatatttg 38

<210> 9

<211> 34

<212> DNA

<213> Homo sapiens

<400> 9
cacaagctta ttacaggatg acattcctct tgtc 34

<210> 10

<211> 51

<212> DNA

<213> Homo sapiens

<400> 10
ttaggcttag gtggttctgg tggttccggt ggttctggtg gatccggtgg a 51

<210> 11

<211> 52

<212> DNA

<213> Homo sapiens

<400> 11
agcttccacc ggatccacca gaaccaccgg aaccaccaga accacctaag cc 52

<210> 12

<211> 18

<212> DNA

<213> Homo sapiens

<400> 12
gatctctagt tccacgtg 18

<210> 13

<211> 18

<212> DNA

<213> Homo sapiens

<400> 13
gatccacgtg gaactaga 18

<210> 14

<211> 117

<212> DNA

<213> Homo sapiens

<400>  14
gccttaggct taggtggttc tggtggttcc ggtggttctg gtggatctct agttccacgt      60

ggatccggtg gtggaatcac tgatgtccgt cctttgtatt caaggagatt accaaaa        117

<210>  15

<211>  39

<212>  PRT

<213>  Homo sapiens

<400>  15

Ala Leu Gly Leu Gly Gly Ser Gly Gly Ser Gly Gly Ser Gly Gly Ser
1               5                   10                  15

Leu Val Pro Arg Gly Ser Gly Gly Gly Ile Thr Asp Val Arg Pro Leu
            20                  25                  30

Tyr Ser Arg Arg Leu Pro Lys
        35